# EUROPEAN PATENT SPECIFICATION

(11) **EP 0 772 613 B1**
(45) Date of publication and mention of the grant of the patent: **21.11.2001**
(21) Application number: 95926937.4
(22) Date of filing: 18.07.1995
(51) Int. Cl.: C07D 451/04

(54) **PROCESS FOR THE PRODUCTION OF AMINOAZOBICYCLOALKANES FROM OXIMES**
VERFAHREN ZUR HERSTELLUNG VON AMINOAZOBICYCLOALKANEN AUS OXIMEN
PROCEDE DE PRODUCTION D'AMINOAZOBICYCOALCANES A PARTIR D'OXIMES

(30) Priority: 23.07.1994 GB 9414900
(43) Date of publication of application: 14.05.1997
(73) Proprietor: F.HOFFMANN-LA ROCHE AG, 4070 Basel (CH)
(72) Inventor: SMITH, Gillian, E., SmithKline Beecham Pharm., Essex CM19 5AD (GB)
(74) Representative: Witte, Hubert, Dr.
(86) International application number: PCT/EP95/02824
(87) International publication number: WO 96/03401

(56) References cited:
- EP-A- 0 013 138
- EP-A- 0 042 705
- DE-A- 3 322 574
- US-A- 4 432 983
- J. ORG. CHEM., vol. 40, no. 3, 1975 pages 381-382, M. S. NEWMAN, V. LEE 'Improved Procedures for Ethynylcarbinol Hydration and Oxime Reduction to Amino Alcohols'
- J. MOL. STRUCT., vol. 172, 1988 pages 381-394, N. CABEZAS ET AL. 'SYNTHESIS AND STRUCTURAL STUDY OF N-(8-ISOPROPYLNORTROPAN-3-alpha-YL)-2-METH OXY-4-AMINO-5-CHLOROBENZAMIDE'

## Description

The present invention relates to a process for preparing *endo*-3-amino-8-methyl-8-azabicylco[3.2.1]octane or *endo*-3-amino-9-methyl-9-azabicylco[3.3.1]nonane which are intermediates to pharmaceutically active compounds.

EP-A-200444 and EP-A-247266 (Beecham Group plc) describe certain compounds which are granatane and tropane derivatives having 5-HT₃ receptor antagonist activity which are described as possessing a number of potential therapeutic utilities, including *inter alia* the treatment of cytotoxic agent induced emesis. Example 6 of EP-A-200444 describes the preparation of granisetron, which is a granatane derivative and Example 5 of EP-A-247266 describes the preparation of BRL 46470A, which is a tropane derivative. The method described in EP-A-247266 for preparing the tropane side chain intermediate results in the formation of a considerable amount of the unwanted *exo* product.

GB 2125398 (Sandoz Limited) describes the preparation of granatyl amines from the corresponding oxime using alane. The use of alane on a large industrial scale is disadvantageous, because it is usually generated by the addition of concentrated sulphuric acid to lithium aluminium hydride, and is a potentially hazardous reducing agent, requiring special precautionary measures.

DE-A-3322574 discloses methods of making endo-9-methyl-9-azabicyclo[3.3.1]non-3-yl amine and exo-8-methyl-8-azabicyclo[3.2.1]octan-3-yl amine by treating the corresponding oximes with lithium aluminium hydride in tetrahydrofurane at -10°C. US-A-4432983 discloses the catalytic hydrogenation of 8-benzyl-1αH,5αH-nortropane-3-one oxime to give the corresponding axial amine. EP-A-0042705 discloses the reduction of tropinone oxime to 3β-amino-8-methyl-8-azabicyclo[3.2.1]octane using sodium in anhydrous amyl alcohol. EP-A-0013138 discloses the hydrogenation of 9-benzyl-9-azabicyclo-[3.3.1]nonan-3-one oxime in the presence of Raney nickel to give 3 alpha-amino-9-benzyl-9-azabicyclo[3.3.1]nonane with a yield of 61%.

J Org Chem, 40 (3), 1975, 381 discloses an improved preparation of certain amino alcohols from ethynyl carbinols. The preparation involves the reduction of hydroxy oximes over a rhodium catalyst.

It is an object of the present invention to provide a new process for preparing *endo*-3-amino-8-methyl-8-azabicylco[3.2.1]octane or *endo*-3-amino-9-methyl-9-azabicylco[3.3.1]nonane, which process is convenient to use on an industrial scale, has a rapid reaction rate and results in a high ratio of desired *endo* product.

Accordingly, the present invention provides a process for preparing a compound of formula (I): 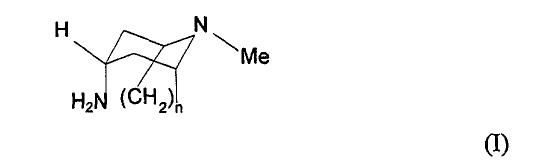 wherein n is 2 or 3;
characterised by reducing a compound of formula (II): 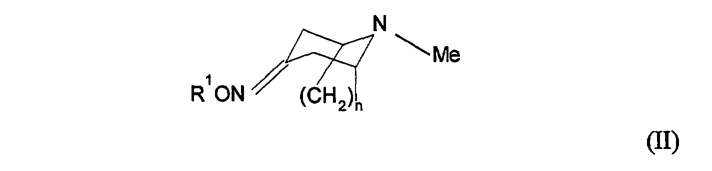 wherein R¹ is hydrogen or C₁₋₄ alkyl, and n is 2 or 3;
by catalytic hydrogenation in the presence of a rhodium catalyst, at an elevated pressure of 20-50 psi (137.9 to 344.8kPa) and an elevated temperature of 25 to 70°C.

Examples of R¹ when C₁₋₄ alkyl include methyl, ethyl, propyl and butyl, in all possible isomers. Preferably R¹ is hydrogen or methyl.

Preferably n is 2.

The catalytic hydrogenation is normally carried out in an organic solvent such as dry methanol, industrial methylated spirits, ethanol and isopropanol or an aqueous/organic solvent mixture such as in aqueous methanol, industrial methylated spirits, ethanol and isopropanol. The reaction is preferably conducted at an elevated temperature of about 50°C and an elevated pressure of 25 to 50 psi (172.4 to 344.8kPa). The rhodium catalyst is usually used on a conventional support medium such as carbon.

The percentage of rhodium to carbon is usually 1 to 10% by weight, preferably around 5%. The catalyst is usually used in a proportion of 1 to 20% by weight of the starting material, preferably at about 10%.

The reaction is optionally carried out in the presence of ammonia which may be added as an aqueous solution or as dry ammonia gas. The addition of ammonia gives a faster, cleaner reaction and reduces the formation of side products. Ammonia is usually added at a molar ratio of 2:1 to 15:1 to the starting material, preferably at a molar ratio of about 9:1.

Compounds of formula (II) are prepared according to conventional procedures, such as those described in Descriptions 1, 2 and 3.

The following Examples illustrate the present invention.

### Description 1

### Preparation of O-methyl-8-methyl-8-azabicyclo[3.2.1]octan-3-one oxime

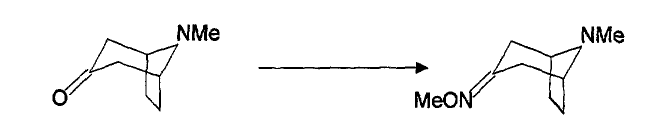

A mixture of 8-methyl-8-azabicyclo[3.2.1]octan-3-one (tropinone) (98.7g, 0.71mole), O-methylhydroxylamine hydrochloride (71.0g, 0.85mole) and water (215ml) was stirred at ambient temperature for 0.5h. The clear solution was basified to pH 12 with 40% aqueous NaOH and extracted with toluene (3x400ml). The combined organic phase was dried (K₂CO₃), filtered and the solvent removed *in vacuo.* The residue was purified by fractional distillation under reduced pressure to afford the ***title compound*** as a colourless oil, 111.1g (93%).
¹H NMR (CDCl₃, JEOL 270MHz): δ1.50 (m,2H), δ2.05 (m,4H), δ2.37 (s,3H), δ2.55 δ(m,1H), δ3.27 (m, 2H) and δ3.80 (s, 3H).
Mass Spec (JEOL DX 303) CI; m/z 168 (M⁺), 137, 96, 82, 42.

### Description 2

### Preparation of 8-methyl-8-azabicyclo[3.2.1]octan-3-one oxime

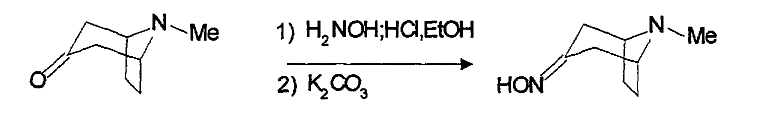

A solution of 8-methyl-8-azabicyclo[3.2.1]octan-3-one (tropinone) (2.0kg) in industrial methylated spirits (IMS) (9.0L) was treated portion-wise with hydroxylamine hydrochloride (1.2kg). The resulting suspension was stirred and heated at reflux for 2 hours then allowed to cool to ambient temperature. The product, 8-methyl-8-azabicyclo[3.2.1]octan-3-one oxime hydrochloride, was filtered, washed with IMS (1.0L) and dried in air.

The hydrochloride salt obtained above was suspended in water (11.5L) and dichloromethane (5.0L) and treated with potassium carbonate (3.9kg). The organic layer was separated and the aqueous layer was extracted twice with dichloromethane (3.0L and 2.0L). The combined organic extracts were dried with potassium carbonate, filtered through CELITE and the solvent evaporated. The residue was triturated with ethyl acetate (2.0L), filtered and dried under vacuum to give the ***title compound*** (1.93kg, 87%), as a white solid
¹H NMR (CDCl₃, JEOL 270MH_{z}): δ11.1 (br s,1H), δ3.3 (m,2H), δ3.0 (d, 1H), δ2.65 (dd, 1H), δ2.4 (s, 3H), δ2.25 (dd, 1H), δ2.15 (d, 1H), δ2.0 (m, 2H), δ1.55 (m, 2H).
¹³C NMR (CDCl₃, JEOL 67.8MHz): δ26.1(CH₂), δ27.0 (CH₂), δ30.8 (CH₂), δ36.8 (CH₂), δ38.8 (CH₂), δ59.8 (CH), δ60.5 (CH), δ154.5 (quat), Mass Spec (JEOL DX303) EI: m/z 154, (M⁺) 137, 96, 82, 42

### Description 3

### Preparation of 9-methyl-9-azabicyclo[3.3.1]nonan-3-one oxime

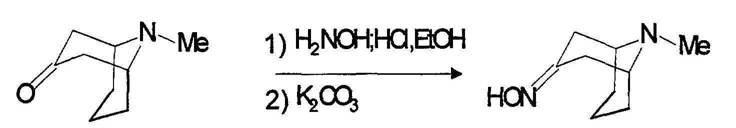

9-Methyl-9-azabicyclo[3.3.1]nonan-3-one (77g,: Org. Synth Coll Vol. 4, 816) was added to a suspension of hydroxylamine hydrochloride (41.7g) in ethanol (350mls). The mixture was heated at reflux for 2 hours, then allowed to cool to ambient temperature. The product solid was filtered, washed with ethanol (50 ml) then diethylether (200ml) and dried in air.

The hydrochloride salt obtained above was suspended in water (390ml and dichloromethane (110ml) and treated with potassium carbonate (49.1g). The organic layer was separated and the aqueous phase was extracted twice with dichloromethane (2x110ml).

The combined organic extracts were dried with potassium carbonate whilst stirring with activated charcoal.

The mixture was filtered through CELITE and the solvent removed by evaporation under reduced pressure to give the crude free base (weight yield = 34.3g).

The crude free base was recrystallised from hot ethyl acetate (144ml) to give the ***title compound*** as a pale yellow solid (26g, 31%).
¹H NMR (CDCl₃, JEOL 270MHz) : δ9.62 (br s,1H), δ3.1 (m,2H), δ3.0 (s,1H), δ2.75 (dd,1H), δ2.55 (s,3H), δ2.4 (dd,1H), δ2.23 (d, 1H), δ1.95 (m,2H), δ1.75 (m,1H) δ1.5 (m,3H).

### Example 1

### Preparation of endo-3-amino-8-methyl-8-azabicyclo[3.2.1]octane

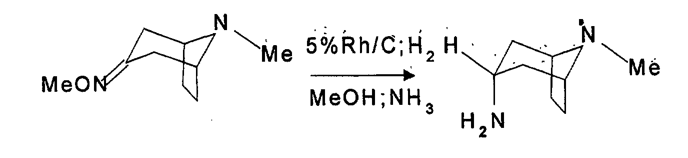

A solution of O-methyl-8-methyl-8-azabicyclo[3.2.1]octan-3-one oxime(500g; 3moles) in methanol (3.2L) and 0.88 ammonia (1.8L; 9.equivs) was hydrogenated over 5% rhodium on carbon paste (50g dry wt.) at 35-50 psi (241.3 - 344.8 kPa) and 50°C for 16 hours. After cooling and purging with nitrogen, the catalyst was filtered off and washed with fresh methanol (2x500ml). The solvent was distilled out and the residue diluted with isopropanol (1.5L). The solvent was again distilled out to leave the title compound as an oil which was distilled (bp 86°/7mm) to give the purified ***title compound*** as a colourless solid (344g, 82%).
¹H NMR (CDCl₃, JEOL 270MHz) : δ3.2 (t,1H), δ3.1 (m,2H), δ2.25 (s,3H), δ2.1 (m,2H), δ2.0 (m,4H) δ1.45 (dd,2H).
¹³C NMR (CDCl₃; JEOL 67.8MHz): δ26.2 (CH₂); δ39.4 (CH₂); δ40.4 (CH or CH₃); δ42.7 (CH or CH₃); δ60.3 (CH or CH₃)
Mass Spec. (JEOL DX303) EI; m/z 140 (M⁺), 124, 96, 83

### Example 2

### Preparation of endo-3-amino-8-methyl-8-azabicyclo[3.2.1]octane

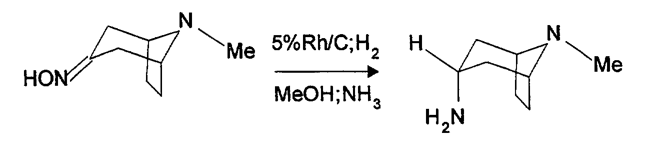

A solution of 8-methyl-8-azabicyclo[3.2.1]octan-3-one oxime (1500g; 9.7moles) in methanol (8.2L) and 0.88 ammonia (5.4L 9.equivs) was hydrogenated over 5% rhodium on carbon paste (50g dry wt.) at 29-44 psi (199.8 - 303.4 kPa) and 50° for 17 hours. After cooling and purging with nitrogen, the catalyst was filtered off and washed with fresh methanol (2x1.5L). The solvent was distilled out and the residue diluted with isopropanol (4L). The solvent was again distilled out to leave the ***title compound*** as an oil which was distilled (bp 68-70°/5mm) to give the purified title compound as a colourless solid (1.23Kg, 90%).
¹H NMR (CDCl₃, JEOL 270MHz): δ3.2 (t,1H), δ3.1 (m,2H), δ2.25 (s,3H), δ2.1 (m,2H), δ2.0 (m,4H) δ1.45 (dd,2H).
¹³C NMR(CDCl₃; JEOL 67.8MHz): δ26.2 (CH₂); δ39.4 (CH₂); δ40.4 (CH/CH₃); δ42.7 (CH/CH₃); δ60.3 (CH/CH₃)
Mass Spec. (JEOL DX303) EI; m/z 140 (M⁺), 124, 96, 83

### Example 3

### Preparation of endo-3-amino-9-methyl-9-azabicyclo[3.3.1]nonane

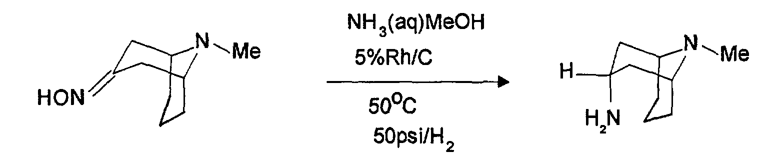

A solution of 9-methyl-9-azabicyclo[3.3.1]nonan-3-one oxime (10g, 0.06 moles) in methanol (70ml) and 0.88 ammonia; (36ml; 10.0 equivs) was hydrogenated at 50°C and 50 psi (344.8kPa)/H₂ for 16 hrs using 5% Rhodium on carbon paste (1g dry weight). The reaction mixture was cooled to room temperature and filtered to remove catalyst. The catalyst bed was washed with methanol and the methanolic solution was then evaporated under reduced pressure to give the ***title compound*** as an oil (8.6g, 93% weight recovery) which solidified to a waxy solid on standing (8.6g, 93%).
G.C./Mass Spec. (JEOL DX303) EI: M/z 154 (M⁺)

## Claims

1. A process for preparing a compound of formula (I): 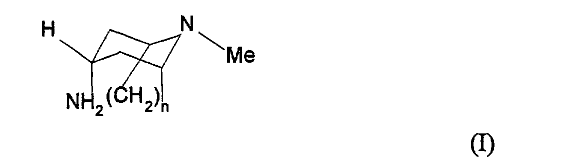 wherein n is 2 or 3;
**characterised by** reducing a compound of formula (II): 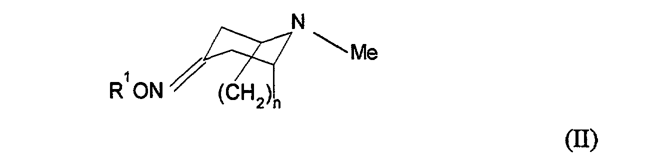 wherein R¹ is hydrogen or C₁₋₄ alkyl and n is 2 or 3;
by catalytic hydrogenation in the presence of a rhodium catalyst, at an elevated pressure of 20-50 psi (137.9 to 344.8kPa) and an elevated temperature of 25 to 70°C.

2. A process according to claim 1 wherein n is 3.

3. A process according to claim 1 wherein n is 2.

4. A process according to claim 1, 2 or 3 wherein R¹ is hydrogen or methyl.

5. A process according to any preceding claim carried out at about 50°C and at 25 to 50 psi (172.4 to 344.8kPa).

6. A process according to any one of claims 1 to 5 wherein the rhodium catalyst is supported on carbon.

7. A process according to any one of claims 1 to 6 wherein the reaction is carried out in the presence of ammonia.

8. A process for the preparation of an amide derivative of compound of a compound of formula (I), which process comprises:
i) preparing a compound of formula (I) as defined in any one of claims 1 to 7; and
ii) reacting the compound of formula (I) with an appropriate carboxylic acid or reactive derivative thereof.

9. A process according to claim 8 for the preparation of granisetron (including pharmaceutically acceptable salts thereof) which process comprises:
i) preparing a compound of formula (I) wherein n is 3; and
ii) reacting the compound of formula (I) with 1-methylindazole-3-carboxylic acid or a reactive derivative thereof;
and optionally forming a pharmaceutically acceptable salt thereof.

## Patentansprüche

1. Ein Verfahren zur Herstellung einer Verbindung mit der Formel (I): 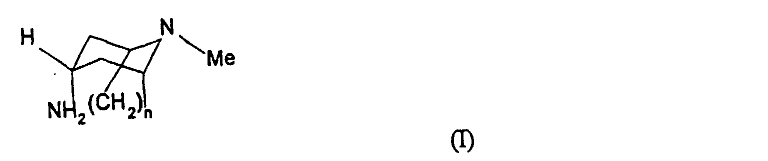 wobei n =2 oder 3;
hergestellt durch die Reduktion einer Verbindung mit der Formel (II): 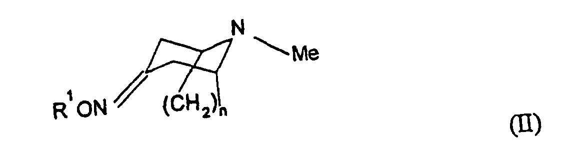 wobei R¹ ein Wasserstoffatom oder einen C₁₋₄ -Alkyl darstellt und n = 2 oder 3; durch katalytische Hydrierung in Gegenwart eines Rhodiumkatalysators, bei einem erhöhten Druck von 20-50 psi (137.9-344.8 kPa) und einer erhöhten Temperatur von 25-70°C.

2. Ein Verfahren gemäss Anspruch 1, wobei n=3.

3. Ein Verfahren gemäss Anspruch 1, wobei n=2.

4. Ein Verfahren gemäss Ansprüche 1, 2 oder 3, wobei R¹ ein Wasserstoffatom oder eine Methylgruppe ist.

5. Ein Verfahren gemäss irgendeiner der oben erwähnten Ansprüche, ausgeführt bei ca. 50°C und 25-50 psi (172.4-344.8 kPa).

6. Ein Verfahren gemäss einer der Ansprüche 1-5, wobei Kohlenstoff das Trägermaterial des Rhodiumkatalysators darstellt.

7. Ein Verfahren gemäss einer der Ansprüche 1-6, wobei die Reaktion in Gegenwart von Ammoniak durchgeführt wird.

8. Ein Verfahren für die Herstellung eines Amid-Derivates einer Verbindung der Formel (I), wobei das Verfahren Folgendes beinhaltet:
i) Herstellung einer Verbindung der Formel (I) gemäss einer der Forderungen 1-7; und
ii) Reaktion der Verbindung mit der Formel (I) mit einer geeigneten Carbonsäure oder einem reaktiven Derivat davon.

9. Ein Verfahren gemäss Anspruch 8 für die Herstellung von Granisetron (inklusive pharmazeutisch verträglicher Salze davon), wobei das Verfahren Folgendes beinhaltet:
i) Herstellung einer Verbindung der Formel (I), wobei n=3; und
ii) Reaktion der Verbindung mit der Formel (I) mit 1-Methylindazol-3-Carbonsäure oder einem reaktiven Derivat hiervon;
und die optionale Herstellung eines pharmazeutisch verträglichen Salzes davon.

## Revendications

1. Un procédé de préparation d'un composé de formule (I) 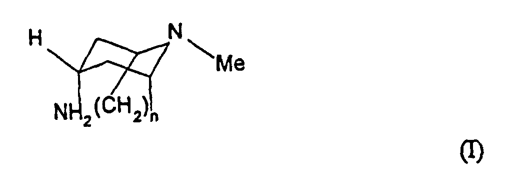 où n = 2 ou 3 ;
**caractérisé par** la réduction d'un composé de formule (II) 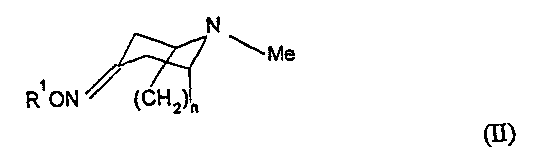 où R¹ est hydrogène ou C₁₋₄ alkyle et n = 2 ou 3 ;
par hydrogénation catalytique en présence d'un catalyseur rhodium, à une pression élevée de 137,9 à 344,8 kPa (20 - 50 psi) et à la température élevée de 25 à 70 °C.

2. Procédé selon la revendication 1 pour lequel n est égal à 3.

3. Procédé selon la revendication 1 pour lequel n est égal à 2.

4. Procédé selon l'une des revendications 1 à 3, pour lequel R¹ est hydrogène ou méthyle.

5. Procédé selon l'une des revendications 1 à 4, pour lequel la température est environ égale à 50 °C et la pression de 172,4 à 344,8 kPa (25 - 50 psi).

6. Procédé selon l'une des revendications 1 à 5, pour lequel le catalyseur rhodium repose sur du carbone.

7. Procédé selon l'une des revendications 1 à 6, pour lequel la réaction se déroule en présence d'ammoniaque.

8. Procédé pour la préparation d'un amide dérivé d'un composé de formule (I), comprenant:
i) la préparation d'un composé de formule (I) tel que défini dans l'une quelconque des revendications 1 à 7 et
ii) la réaction du composé de formule (I) avec un acide carboxylique approprié ou avec un réactif dérivé de cet acide.

9. Procédé selon la revendication 8 pour la préparation du granisétron (y compris ses sels acceptables d'un point de vue pharmaceutique), comprenant :
i) la préparation d'un composé de formule (I) où n = 3 et
ii) la réaction du composé de formule (I) avec l'acide 1-méthylindazole-3-carboxylique ou avec un réactif dérivé de cet acide
et, le cas échéant, formant un sel de cet acide acceptable d'un point de vue pharmaceutique.
